(19) Europäisches Patentamt
European Patent Office
Office européen des brevets

(11) Publication number: 0 041 366 B1

(12) EUROPEAN PATENT SPECIFICATION

(45) Date of publication of patent specification: 10.09.86

(51) Int. Cl.[4]: G 01 N 35/02, G 01 N 33/52, G 01 N 21/27

(21) Application number: 81302354.6

(22) Date of filing: 28.05.81

# (54) Method for operating an apparatus for analysing samples optically.

(30) Priority: 30.05.80 JP 71374/80
03.09.80 JP 121093/80

(43) Date of publication of application:
09.12.81 Bulletin 81/49

(45) Publication of the grant of the patent:
10.09.86 Bulletin 86/37

(84) Designated Contracting States:
DE FR GB SE

(56) References cited:
FR-A-2 097 329
NL-A-7 709 175
US-A-3 621 215
US-A-3 838 010
US-A-4 063 816
US-A-4 155 978

(73) Proprietor: Hitachi, Ltd.
5-1, Marunouchi 1-chome
Chiyoda-ku Tokyo 100 (JP)

(72) Inventor: Furuta, Yoshiteru
663 Ichige
Katsuta-shi (JP)
Inventor: Nomura, Yasushi
991-15 Motoyoshidacho
Mito-shi (JP)

(74) Representative: Paget, Hugh Charles Edward et al
MEWBURN ELLIS & CO. 2/3 Cursitor Street
London EC4A 1BQ (GB)

(56) References cited:
CLINICAL CHEMISTRY, vol. 18, no. 12, 1972
D.W. MOSS: "The Relative Merits and Applicability of Kinetic and Fixed-Incubation Methods of Enzyme Assay in Clinical Enzymology" pages 1449-1454
Methoden der Enzymatischen Analyse, 3. Auflage, 1974, Verlag Chemie GmbH, Weinheim/Bergstrasse, Seiten 111-128;150-152;1491-1496;2138-2146

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 041 366 B1

**Description**

This invention relates to a method of operating an apparatus for analysing samples optically each with respect to a plurality of items.

In the quantitative analysis of a sample containing many components, particularly that of a metabolic material in body fluid such as blood, analytical methods utilizing an enzyme which acts specifically on a metabolic material have been recently employed. An enzyme reaction proceeds under very mild conditions in a short time. Enzymes have a property of acting merely on a specific material even if it contains many contaminants. Analytical methods utilizing an enzyme reaction having such advantages are employed for biochemical inspection in hospitals, etc.

Conventional photometric methods utilizing an enzyme reaction are generally directed to quantitative analysis of only one anaylsis item in one sample placed in one reaction vessel, as disclosed, for example, in Patent No. US—A—3,838,010.

An apparatus is known from US—A—4,155,978 which has a turnable with a plurality of reaction vessels arranged in line. The pitch spacing of the vessels is such that, for a position initially occupied by one of the vessels, any other vessel may be moved into that position by an appropriate rotation of the turntable. There is a first dispenser for charging a reactor vessel with a first reagent at a first such position, a second dispenser for charging said reactor vessel with a second reagent, means for detecting a light beam passing through a said reactor vessel at a second such position to obtain a measurement of light absorption, and a sample-feeding means for feeding a sample to a said reactor vessel at a third such position. This document therefore corresponds to the pre-characterising part of claim 1.

In the apparatus of US—A—4,155,978, however, the method of operation involves the control of the disc so that it rotates by one pitch each time it rotates. Therefore the vessels are sequentially moved into the position in which the light beam is passed through them and stopped in that position. The light beam is activated only when the vessels are at rest.

It has been found that this is unsatisfactory, and therefore the present invention proposes a method of operating an apparatus of the above mentioned kind characterised in that said first reagent is a first enzyme reagent, said second reagent is a second enzyme reagent, said reactor vessel being charged therewith at a fourth such position that the apparatus is controlled so as to obtain, for a sample in a given said reactor vessel, the said analysis with respect to the said plurality of items from an output of the light beam detecting means after said reactor vessel is charged with the the first enzyme reagent is charged to the and an output of the light beam detecting means after the second enzyme reagent is charged to the said given reactor vessel;

the method comprising and in that there are performed the repeated alternating steps of

actuating the first dispenser, the second dispenser, and the sample feeding means while the turntable is at rest, and

rotating the turntable by 360° plus or minus one pitch so that each reactor vessel occupies the second such position in turn and stopping the turntable after the rotation so that each said reactor vessel comes to rest one pitch away from its previous rest position, the light beam detecting means being actuated during the step. Thus during rotation all the vessels pass through the light beam, although the final position of each vessel only moves by one pitch. Furthermore, in the present invention the second dispenser changes the second reagent in a fourth vessel position, and not the same position as the charging of the first reagent in US—A—4,155,978.

It has been proposed, in NL—A—7709175, to perform two analyses on a single sample, provided that the optical characteristics used to detect the two analysis reactions do not interfere with each other. This proviso restricts the choice of reagents used in the analyses. With the method of the present invention this restriction is not necessary as the reagents are required to be added sequentially, not simultaneously, the first analysis is performed with a measurement taken before the addition of the second reagent, and, in performing the second analysis, a measurement taken after the addition of the second reagent is compared with a measurement taken after the addition of the first reagent. NL—A—7709175 does propose the sequential addition of reagents, but no reason is given for this except for reference to an incubation period. It does not propose the taking of any measurements between the respective additions of the reagents.

One advantage obtainable in embodiments of the present invention is that only a very small amount of a sample may be enough for the analysis of a plurality of items.

It is also possible to provide an efficient analytical method where sampling number can be decreased, so that second or successive sampling operations can be omitted for further analysis items.

Furthermore it is possible for a plurality of enzyme reactions to be utilized for analyzing a plurality of items, and thus the reaction of the first analysis item does not interfere with the reaction of the second analysis item, resulting in very small error in measurement.

An embodiment of the present invention will now be discussed in detail, by way of example, with reference to the accompanying drawings, in which:

Fig. 1 is a diagram schematically showing a structure of one embodiment according to the present invention,

Fig. 2 is a diagram showing a signal-processing system of the embodiment of Fig. 1.

Fig. 3 is a diagram showing the measurement of three analysis items in one sample,

Fig. 4 is a diagram showing a reaction process in the analysis of lactic acid dehydroenzyme (LDH) and leucine aminopeptidase (LAP).

Fig. 5 is a diagram showing a reaction process in the analysis of glumatic oxalacetic transaminase (GOT) and glutamic pyruvic transaminase (GPT).

Several examples of applying an enzymatic analytical method to a serum sample will be described below in connection with the present invention.

Example 1:

Glucose in serum undergoes the following reaction:

$$\text{Glucose}+0_2 \xrightarrow{\text{Glucose oxidase}} \text{Gluconic acid}+H_2O_2$$

Cholesterol includes an ester form and a free form, and each form undergoes the following reaction. Whole cholesterol is the total of the two forms:

$$\text{Ester form cholesterol}+H_2O \xrightarrow{\text{Cholesterol esterase}} \text{Free form cholesterol}+\text{Fatty acid} \quad (2)$$

$$\text{Free form cholesterol}+O_2 \xrightarrow{\text{Cholesterol oxidase}} \Delta^4\text{-Cholestenone}+H_2O_2 \quad (3)$$

Neutral fat undergoes the following reaction:

$$\text{Neutral fat}+3H_2O \xrightarrow{\text{Lipoprotein Lipase}} \text{Glycerol}+\text{Fatty acid} \quad (4)$$

$$\text{Glycerol}+O_2 \xrightarrow{\text{Glycerol oxidase}} \text{Glyceraldehyde}+H_2O_2 \quad (5)$$

Phospholipids undergo the following reaction:

$$\text{Phospilipid} \xrightarrow{\text{Phosphilipase D}} \text{Choline} \quad (6)$$

$$\text{Choline}+O_2 \xrightarrow{\text{Choline oxidase}} \text{Betaine}+H_2O_2 \quad (7)$$

Hydrogen peroxide ($H_2O_2$) produced in the above-mentioned reactions according to formulae (1), (3), (5) and (7) undergoes reaction according to the following formula (8) by action of peroxidase to produce a red pigment, and thus the reaction can be traced by monitoring by photometer.

$$H_2O_2+\text{4-Aminoantipyrine}+\text{Phenol} \xrightarrow{\text{Peroxidase}} \text{Red quinone} \quad (8)$$

The present invention will be described below, referring to Fig. 1.

A reaction disc 1 has, on its circumferential edge, a plurality of (for example 40) light-transmitting reactor vessels 2 serving also as measuring cells, and can be rotated clockwise either by one full turn or by divisional pitch-by-pitch turn around rotary shaft 3.

Sample table 4 has a plurality of sample containers 5 on its circumferential edge, and can be intermittently rotated clockwise step by step around rotary shaft 6. Pipetting of a sample is carried out by pipette 7 provided with sampling probe 8, and the first and second enzyme reagents are poured into the vessels portion by portion by metering pumps 9 and 10. Photometer 11 is of the same multi-wavelength photometer type having a plurality of detectors as that of photometer 63 shown in Fig. 1, and arranged to face light source lamp 12 through a line of the reactor vessels so that light beam 13 from the light source can pass through the lines of reactor vessels 2, while the reaction disc is in rotation.

When the reaction disc 1 is at rest, the light beam 13 of the photometer passes through the center of one reactor vessel, for example, at the 31st position as counted clockwise from the sample-discharge position <u>25</u>, to that reactor vessel. In the following description positions on the disc will be referred to by underlined numbers. A plurality of solution-discharge pipes 26 and a plurality of washing water-discharge pipes 27 are provided between the position of light beam 13 and sample-discharge position <u>25</u> so that the pipes can be inserted into or removed, from the reactor vessels. The pipes are also connected to a solution-discharging device 28 and a washing device 29, respectively.

The whole structure of electric-signal-processing system 40 has as shown in Fig. 2 of multiplexer 14,

logarithm conversion amplifier 15, A/D converter 16, central processing unit 17, reading-out memory (ROM) 18, read-out and write memory (RAM) 19, printer 20, operating panel 21 and mechanism-driving circuit. They are connected to bus line 23.

Next, we will describe the operation of this embodiment. When sample container 5 containing a sample to be analysed, such as serum, arrives at sampling position 30, the tip end of probe (suction-and-discharge pipe) 8 of pipette 7 is inserted into sample container 5, and a predetermined amount of serum is taken up by suction and retained inside probe 8. Thereafter, probe 8 moves to discharging position 25 on reaction table 1, and then charges the serum retained therein into reactor vessel 2 at sample-receiving position 25. When the sampling operation is completed, reaction disc 1 is actuated to rotate clockwise continuously or intermittently until all the reactor vessels 2 on reaction disc 1 plus the first reaction vessel again passes through discharge position 25, so the disc 1 rotates by 369°.

The rotation of reaction disc 1 causes reactor vessel 2 containing the sample taken up by sampling operation to stop at a position displaced only by one pitch, that is, only by 9°, from the discharge position 25, that is, the position 31 at which the first reagent is added. During the rotation of reaction disc 1, all of reactor vessels 2 on reaction disc 1 pass across light beam 13. When each of reactor vessels 2 passes through light beam 13, light absorption measurement of each sample solution is carried out by spectroscope 11. From the output of spectroscope 11, signals with wavelength now necessary for the measurement are selected by multiplexer 14, and then put into central processing unit 17 through A/D converter 16, and memorized in reading-and-writing memory 19.

Suppose that the period for rotation and rest of reaction disc 1 be, for example, 30 seconds. Operation and rest for the 30 seconds is repeated as one cycle. With repetitions of the cycle, a specific sample taken up can take a clockwise one pitch advanced position when reaction disc 1 is at rest.

Metering pump 9 is directed to introducing the first enzyme reagent solution in tank 35 into reactor vessels, and metering pump 10 is directed to introducing the second enzyme reagent solution in tank 36 into reactor vessels. The first and second enzyme reagent solutions may have the following compositions:

Composition of first enzyme reagent solution:

| | |
|---|---|
| Phosphate buffer (pH 7.0) | 100m mole/liter |
| Glucose oxidase | 18U/ml |
| Peroxidase | 1.2U/ml |
| 4-Aminoantipyrine | 0.8m mole/ml |
| Phenol | 11m mole/liter |

Composition of second enzyme reagent solution:

| | |
|---|---|
| Phosphate buffer (pH 7.7) | 5 moles/liter |
| Cholesterol esterase | 2U/liter |
| Cholesterol oxidase | 3U/liter |
| Methanol | 10 moles/liter |
| Hydroxypolyethoxydodecane | 4% |

The discharge pipes 33 and 34 of metering pumps 9 and 10, respectively, are vertically movable, and descend a little when the reagent solutions are discharged. Discharge pipe 33 of metering pump 9 and discharge pipe 33 of metering pump 10 are provided over reactor vessel 2 at reagent-adding position 31, that is, the 1st position counted clockwise from discharge position 25, and over reactor vessel 2 at reagent-adding position 32, that is, the 16th position counted clockwise from discharge position 25, respectively, for example, when reaction disc 1 is at rest. That is, a given sample in reactor vessel 2 is admixed with the first enzyme reagent at reagent-adding position 31, whereby enzyme reaction of first group is initiated, and when the relevant reactor vessel reaches reagent-adding position 32 at the 15th cycle, the second enzyme reagent is added to the reactor vessel by metering pump 10, whereby the second enzyme reaction is initiated. When reactor vessel 2 moves its position at the rest of reaction disc 1 across light beam 13 to between light beam 13 and sample-receiving position 25 with further repetitions of the cycle, measurement of the given sample in the reactor vessel can be regarded as completed, and the given sample solution is discharged by suction through discharge pipe 26 by discharging device 28. Subsquently, washing water (usually distilled water) is charged into the reactor vessel through wash water discharge pipe 27 from washing device 29. At the subsequent rest of reaction disc 1, the washing water is discharged from the reactor vessel in the same manner as above, and the washed reactor vessel is reused for another sample at sample-receiving position 25. This cycle is then repeated as often as necessary. These operations

are carried out under control of the central processing unit 17 through mechanism part-diving circuit 22 according to the program of read-out memory 18. Operating panel 21 is used for such operation as input of measuring conditions, start and discontinuation of measurement, etc.

Suppose that reaction disc 1 have a rest time of 9.5 seconds and a rotation time of 20.5 seconds in one cycle of the foregoing operation. Reaction progress of the given sample can be measured 31 times at intervals of 29.5 seconds, and thus data resulting from the measurements for 15 minutes 15 seconds are memorized in read-out and write memory 19. Central processing unit 17 operates according to the program of read-out memory 18, extracts the necessary data from 31 measurement data in read-out and write memory 19 according to the predetermined program, and gives output to printer 20 after processing such as concentration calculation, etc.

Description will be made below of some detail referring to an example, where the apparatus according to the embodiment of Fig. 1 is applied to analysis of two items of glucose and whole cholesterol.

On the basis of 31 absorbance data for each sample which have been memorized in read-out and write memory 19, concentration is calculated in the following manner according to predetermined program. That is, suppose that 16th absorbance datum be $E_{16}$ and 31th datum $E_{31}$.

Glucose concentration $Y_1$ will be expressed as follows:

$$Y_1 = \frac{C_S}{E^S_{16} - E^O_{16}} (E_{16} - E^O_{16})$$

Whole cholesterol concentration $Y_2$ will be expressed as follows:

$$Y_2 = \frac{C_S'}{E^S_{31} - {}_K E^S_{16}} (E_{31} - {}_K E_{16})$$

wherein $C_S$ and $C_S'$ are glucose concentration and cholesterol concentration, respectively, of standard solution used for preparing a working curve, and memorized as input from operating panel 21; $E^O_{16}$ is 16th data for the reagent blank; $E^S_{16}$ and $E^S_{31}$ are 16th data for glucose and 31th data for whole cholesterol of standard solution; K is a correction factor for the amount of solution and in this case, K=505/555 because the amount sample is 5µl, that of first enzyme reagent solution 500µl and that of second enzyme reagent solution 50µl.

The present invention is applicable not only to the analysis of two components but also to that of three or more components. For example, in order to analyze three components by use of the apparatus of the embodiment shown in Fig. 1 a third enzyme reagent-adding position may be provided between second reagent-adding position 32 and light beam 13. For example, in analyzing three components of glucose, whole cholesterol and neutral fat, after the above-mentioned analysis of two components of glucose and the whole cholesterol, lipoprotein lipase and glycerol oxidase are added as third reagents to the reaction solution to complete the reactions of formulae (4), (5) and (8), and the concentration of neutral fat is calculated from the difference between the absorbances before and after the addition of the third reagents.

When the absorbence of the reaction solution in this case is traced with time, the results will be as given in Fig. 3. The magnitude of a, b and c in Fig. 3 are proportional to the respective concentrations of glucose, whole cholesterol and neutral fat.

I, II and III in Fig. 3 show the points of time of adding the first, second and third enzyme reagents, respectively.

Although in embodiment shown in Fig. 1 light of the same wavelength is used for analysing two components, different wavelengths may be used for analysing the first component and for analysing the second component respectively.

In this case, data with two different wavelengths can be obtained as 16th absorbance datum, or 15th absorbance datum $E_{15}$ may be obtained as data for the first component with a wavelength different from the measuring wavelengths for $E_{16}$ and $E_{31}$.

In this case, concentration $Y_1$ of the first component can be calculated as follows:

$$Y_1 = \frac{C_S}{E^S_{15} - E^O_{15}} (E_{15} - E^O_{15})$$

Concentration $Y_2$ of the second component can be expressed by the following equation:

$$Y_2 = \frac{C_S'}{E^S_{31} - {}_K E^S_{16}} (E_{31} - {}_K E_{16})$$

A few methods of measuring the activity of enzyme contained in a sample by rate assay method will be described below. The analytical apparatus shown in Fig. 1 will be used in the following methods.

Example 2

The below described method for analyzing two analysis items of lactate dehydrogenase (LDH) and leucine aminopeptidase (LAP) contained in a serum sample is applied to the apparatus in Fig. 1. As examples of suitable reagent compositions in this case, solutions having the following compositions are used, where NADH means reduced form nicotineamide adenine dinucleotide.

Composition of first reagent solution:

| | |
|---|---|
| Pyruvic acid | 0.6m moles/liter |
| Phosphate buffer (pH 7.5) | 50m moles/liter |
| NADH | 0.18m moles/liter |

Composition of second reagent solution:

| | |
|---|---|
| L-leucine-p-nitroanilide | 3.2m moles/liter |
| Phosphate buffer (pH 7.5) | 400m moles/liter |

Measuring conditions for the apparatus are as follows:

| | |
|---|---|
| Amount of sample | 20μl |
| Amount of first reagent | 500μl |
| Amount of second reagent | 250μl |
| Reaction temperature | 25°C |
| Measuring wavelengths 1 | 340nm/376nm |
| Measuring wavelengths 2 | 405nm/505nm |

When the above-mentioned first and second reagents are placed in solution tank 35 for metering pump 9 and solution tank 36 for metering pump 10, respectively, and sample table 4 is loaded with the sample, then an instruction "start analysis" is given from operating panel to actuate the apparatus.

Reaction in reactor vessel 2 is traced. Reaction proceeds according to the following formula (9) from the point of time of mixing the sample with the first reagent solution.

$$\text{Pyruvic acid}+\text{NADH}\xrightarrow{\text{LDH}}\text{Lactic acid}+\text{NAD} \qquad (9)$$

The second reagent is subsequently added thereto after 7.5 minutes, and reaction starts according to the following formula (10) in parallel with the reaction according to the above formula (9).

$$\text{L-leucine-p-nitroanilide}+H_2O\xrightarrow{\text{LAP}}\text{L-leucine}+\text{p-nitroaniline} \qquad (10)$$

The rate of reaction of formula (9) can be determined by tracing the absorbance of NADH according to the single beam dual-method at 340nm/376nm, and is proportional to the activity of LDH. The rate of reaction of formula (10) can be determined by tracing formation rate of p-nitroaniline through the absorbance according to the single beam dual-wavelength method, and is proportional to the activity of LAP.

In the case of the combined use of these two pairs of wavelengths, as shown in Fig. 4, the measuring wavelengths are changed from 340nm/376nm for the measurement of the reaction by the first reagent to 405nm/505nm at the point of time of adding the second reagent. After the change, the components for the reaction of formula (9) contain no such components that substantially absorb the relevant wavelengths, and thus only the reaction of formula (9) can be traced. In Fig. 4, A is the point of time of adding the first reagent. Between the point of time A and the point of time B, LDH reaction takes place, and after the point of time B, LAP reaction takes place.

Example 3

Description will be made below of another method for analyzing two analysis items of glutamic oxalacetic transaminase (GOT) and glutamic pyruvic transaminase. As examples of suitable reagent compositions in this case, solutions of the following compositions can be used.

Composition of first reagent solution:

| | |
|---|---|
| α-Ketoglutaric acid | 18m moles/liter |
| L-aspartic acid | 200m moles/liter |
| NADH | 0.18m moles/liter |
| MDH | ≧0.6U/ml |
| LDH | ≧1.2U/ml |
| Phosphate buffer (pH 7.4) | 80m moles/liter |

Composition of second reagent solution:

| | |
|---|---|
| L-alanine | 6.4m moles/liter |
| Phosphate buffer (pH 7.4) | 80m moles/liter |

The measurement conditions for the apparatus are as follows:

| | |
|---|---|
| Amount of sample | 20μl |
| Amount of first reagent | 350μl |
| Amount of second reagent | 50μl |
| Reaction temperature | 25°C |
| Measurement wavelengths | 340nm/376nm |

When said method is applied to the appartaus in Fig. 1, the reactions of formulae (11) and (12) proceed after the addition of the first reagent.

$$\text{L-aspartic acid}+\alpha\text{-Ketoglutaric acid}\xrightarrow{\text{GOT}}\text{Glutamic acid}+\text{Oxalacetic acid} \quad (11)$$

$$\text{Oxalacetic acid}+\text{NADH}\xrightarrow{\text{MDH}}\text{Malic acid}+\text{MAD} \quad (12)$$

When the second reagent is subsequently added, the reactions of formulae (13) and (9) proceed in parallel with the reactions of formulae (11) and (12).

$$\text{L-alanine}+\alpha\text{-Ketoglutaric acid}\xrightarrow{\text{GPT}}\text{Glutamic acid}+\text{Pyruvic acid} \quad (13)$$

The rate of the reaction of formula (11) is proportional to GOT concentration in the sample and a decreasing rate of NADH in the reaction of formula (12) coupled with the reaction of formula (11), and a decreasing rate of NADH can be determined from the absorbances at 340nm/376nm. That is to say, as shown in Fig. 5, an absorbance change per minute, $X_1$, can be obtained from 15 absorbance data in 15 cycles for 7.5 minutes after the addition of the first reagent, and the activity $Y_1$ of GOT can be obtained as the following formula:

$$Y_1=\frac{X_1\times V_1\times 1{,}000}{\varepsilon\times d\times v} \quad (14)$$

wherein $V_1$ is a total volume of reaction solution (V=370μl); ε is a molecular absorption coefficient (ε=4.20); d is a length of the optical path (d=1cm); and v is a volume of samples (v=20μl).

Thus, $Y_1$ in formula (15) will be as follows:

$$Y_1=X_1\times\frac{370\times1{,}000}{4.20\times1\times20}$$

$$=X_1\times4{,}405 \qquad (15)$$

An absorbance change per minute, $X_2$, can be obtained from 15 absorbance data after the addition of the second reagent and is proportional to the sum ($Y_2$) of the activities of GOT and GPT. That is to say,

$$Y_2=\frac{X_2\times V_2\times1{,}000}{\varepsilon\times d\times v} \qquad (16)$$

and since $V_2=420\mu l$,

$$Y_2=X_2\times\frac{420\times1{,}000}{4.20\times1\times20}$$

$$=X_2\times5{,}000 \qquad (17)$$

Accordingly, the activity $Y_3$ of GPT can be obtained from the following equation:

$$Y_3=Y_2-Y_1$$

In Fig. 5 A is the point of time for adding the first reagent, and B is the point of time for adding the second reagent.

**Claims**

1. A method of operating an apparatus for analysing samples optically each with respect to a plurality of items, which apparatus comprises a turntable (1) provided with a plurality of reactor vessels (2) arranged in line with a pitch spacing so that, for a position initially occupied by one of the said reactor vessels, any other of the said reactor vessels may be moved into that position by an appropriate rotation of the turntable, a first dispenser (9) for charging a reactor vessel with a first reagent at a first such position (31), a second dispenser (10) for charging said reactor vessel with a second reagent, means (11) for detecting a light beam (13) passing through a said reactor vessel (2) at a second such position to obtain a measurement of light absorption, and a sample-feeding means (7, 8) for feeding a sample to a said reactor vessel at a third such position (25), characterised in that:

said first reagent is a first enzyme reagent, said second reagent is a second enzyme reagent, said reactor vessel being charged therewith at a fourth such portion,

and in that the apparatus is controlled so as to obtain, for a sample in a given said reactor vessel (2), the said analysis with respect to the said plurality of items from an output of the light beam detecting means (11) after the said given reactor vessel is charged with the first enzyme reagent and an output of the light beam detecting means (11) after the said reactor vessel (2) is charged with the second enzyme reagent, and in that there are performed the repeated alternating steps of:

(i) actuating the first dispenser (9), the second dispenser (10), and the sample feeding means (7, 8) while the turntable (1) is at rest and

(ii) rotating the turntable (1) by 360° plus or minus one pitch so that each reactor vessel (2) occupies the second such position in turn and stopping the turntable (1) after the rotation so that each said reactor vessel comes to rest one pitch away from its previous rest position, the light beam detecting means (11) being actuated during step (ii).

2. A method according to claim 1 in which as the said light beam detecting means (1) a multi-wavelength photometer is used.

**Patentansprüche**

1. Verfahren zum Betreiben eines Apparatus zur optischen Analyse von Proben jeweils hinsichtlich mehrerer Gesichtspunkte, wobei der Apparat einen Drehtisch (1) mit mehreren Reaktionsgefäßen (2), die mit einem Teilungsabstand derart in Reihe angeordnet sind, daß bei einer von einem der Reaktionsgefäße anfänglich eingenommenen Position jedes andere Reaktionsgefäß durch entsprechende Drehung des Drehtisches in diese Position bewegt werden kann, eine erste Abgabeeinrichtung (9) zum Beschicken eines Reaktionsgefäßes mit einem ersten Reagens an einer ersten derartigen Position (31), eine zweite Abgabeeinrichtung (10) zum Beschicken des Reaktionsgefäßes mit einem zweiten Reagens, eine Einrichtung (10) zum Erfassen eines ein solches Reaktionsgefäß (2) an einer zweiten derartigen Position

durchsetzenden Lichstrahls (13) zur Erzielung einer Messung der Lichtabsorption, und eine Probenzuführeinrichtung (7, 8) zum Zuführen einer Probe an ein solches Reaktionsgefäß an einer dritten derartigen Position (25) aufweist, dadurch gekennzeichnet,

daß das erste Reagens ein erstes Enzym-Reagens und das zweite Reagens ein zweites Enzym-Reagens ist und das Reaktionsgefäß damit an einer vierten derartigen Position beschickt wird,

daß der Apparat derart gesteuert wird, daß für eine Probe in einem gegebenen Reaktionsgefäß (2) die Analyse bezüglich der mehreren Gesichtspunkte aus einem Ausgangssignal der Lichstrahl-Erfassungseinrichtung (11) nach dem Beschicken des gegebenen Reaktionsgefäßes mit dem ersten Enzym-Reagens und einem Ausgangssignal der Lichtstrahl-Erfassungseinrichtung (11) nach dem Beschicken des Reaktionsgefäßes (2) mit dem zweiten Enzym-Reagens gewonnen wird, und

daß die folgenden wiederholten, abwechselnden Schritte durchgeführt werden:

(i) Betätigen der ersten Abgabeeinrichtung (9), der zweiten Abgabeeinrichtung (10) und der Probenzuführeinrichtung (7, 8), während der Drehtisch (1) steht, und

(ii) Drehen des Drehtisches (1) um 360° plus oder minus eine Teilung, so daß jedes Reaktionsgefäß (2) die anschließende zweite derartige Position einnimmt, und Anhalten des Drehtisches (1) nach der Drehung, so daß jedes Reaktionsgefäß eine Teilung von seiner vorherigen Ruheposition entfernt zu stehen kommt, wobei die Lichtstrahl-Erfassungseinrichtung (11) während des Schrittes (ii) betätigt wird.

2. Verfahren nach Anspruch 1, wobei als Lichtstrahl-Erfassungseinrichtung (1) ein Mehrfach-Wellenlängen-Photometer verwendet wird.

**Revendications**

1. Procédé d'utilisation d'un appareil d'analyse optique d'échantillons, chacun en rapport avec une pluralité de sujets, lequel appareil comporte un plateau rotatif (1) muni d'une pluralité d'enceintes réactionnelles (2) qui sont disposées selon une rangée avec un pas d'espacement tel que pour une position initialement occupée par l'une desdites enceintes réactionnelles, n'importe quelle autre de ces enceintes réactionnelles peut être amenée dans cette position au moyen d'une rotation appropriée du plateau rotatif, un premier distributeur (9) servant à introduire un premier réactif dans une enceinte réactionnelle dans une première de ces positions (31), un second distributeur (10) servant à introduire un second réactif dans ladite enceinte réactionnelle, des moyens (11) pour détecter un faisceau de lumière (13) traversant ladite enceinte réactionnelle (2) dans une seconde de ces positions pour obtenir une mesure de l'absorption de la lumière, et des moyens (7,8) d'amenée des échantillons, servant à amener un échantillon jusqu'à ladite enceinte réactionnelle dans une troisième de ces positions (25), caractérisé en ce que:

ledit premier réactif est un premier réactif enzymatique, ledit second réactif est un second réactif enzymatique, lesdits réactifs étant introduits dans ladite enceinte réactionnelle dans une quatrième de ces positions,

et que l'appareil est commandé de manière à fournir, pour un échantillon situé dans ladite enceinte réactionnelle donnée (2), ladite analyse en rapport avec ladite pluralité de sujets à partir d'un signal de sortie des moyens (11) de détection du faisceau de lumière, après que ledit premier réactif enzymatique ait été introduit dans ladite enceinte réactionnelle, et à partir d'un signal de sortie des moyens (11) de détection du faisceau de lumière après que ledit second réactif enzymatique ait été introduit dans ladite enceinte réactionnelle (2), et

que l'on exécute les phases opératoires alternées répétées consistant à:

(i) actionner le premier distributeur (9), le second distributeur (10) et les moyens (7, 8) d'amenée des échantillons, alors que le plateau rotatif (1) est au repos, et

(ii) faire tourner le plateau rotatif (1) de 360° plus ou moins un pas, de sorte que chaque enceinte réactionnelle (2) occupe à son tour ladite seconde position, et à arrêter le plateau rotatif (1) après la rotation de sorte que chacune desdites enceintes réactionnelles vient au repos un pas après sa position de repos antérieure, les moyens (11) de détection du faisceau de lumière étant actionnés pendant la phase opératoire (ii).

2. Procédé selon la revendication 1, selon lequel on utilise, en tant que moyens (11) de détection du faisceau de lumière, un photomètre à longueurs d'onde multiples.

FIG. 1

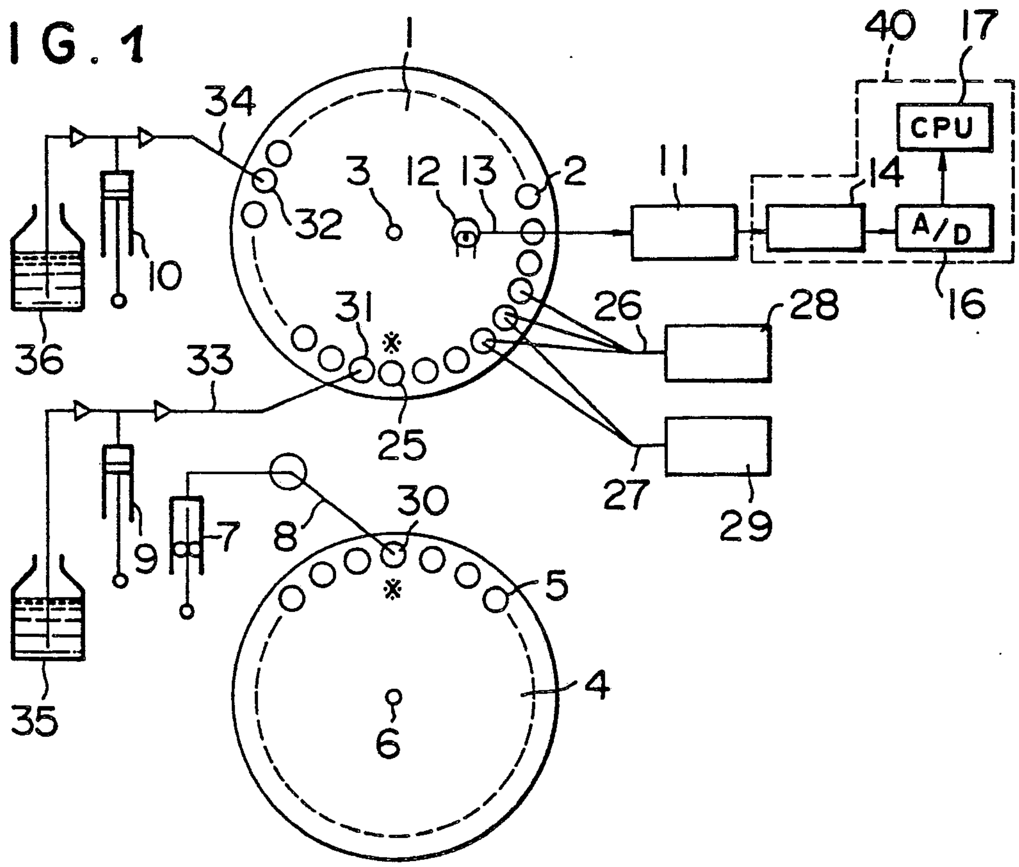
1
2
3
4
5
6
7
8
9
10
11
12
13
14
16
17
25
26
27
28
29
30
31
32
33
34
35
36
40
CPU
A/D

FIG.2

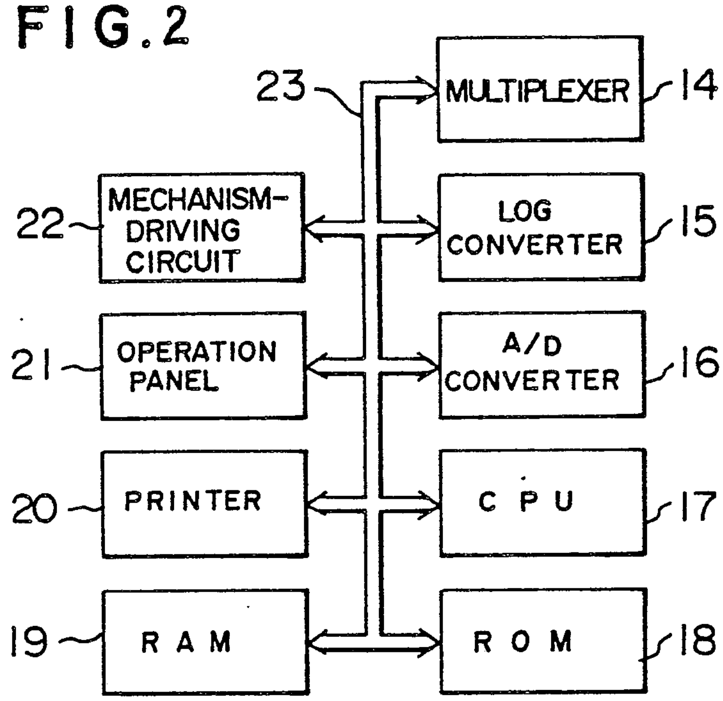
23
MULTIPLEXER
14
22
MECHANISM-DRIVING CIRCUIT
LOG CONVERTER
15
21
OPERATION PANEL
A/D CONVERTER
16
20
PRINTER
C P U
17
19
R A M
R O M
18

FIG.3

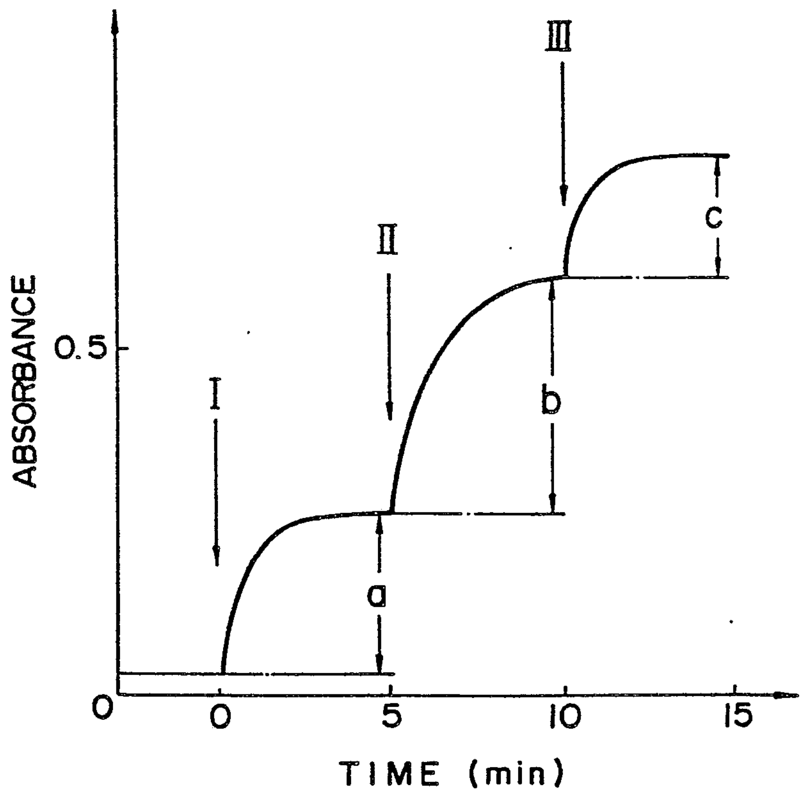
III
II
I
c
b
a
ABSORBANCE
0.5
0
0
5
10
15
TIME (min)

FIG. 4

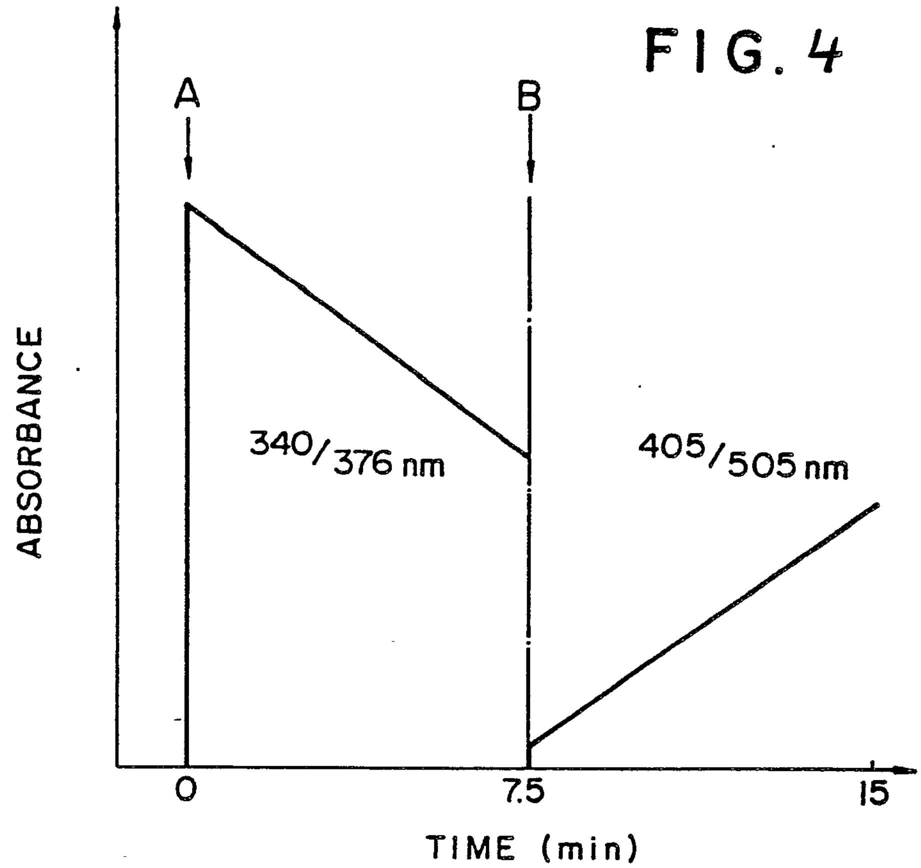
A
B
ABSORBANCE
340/376 nm
405/505 nm
0
7.5
15
TIME (min)

FIG. 5

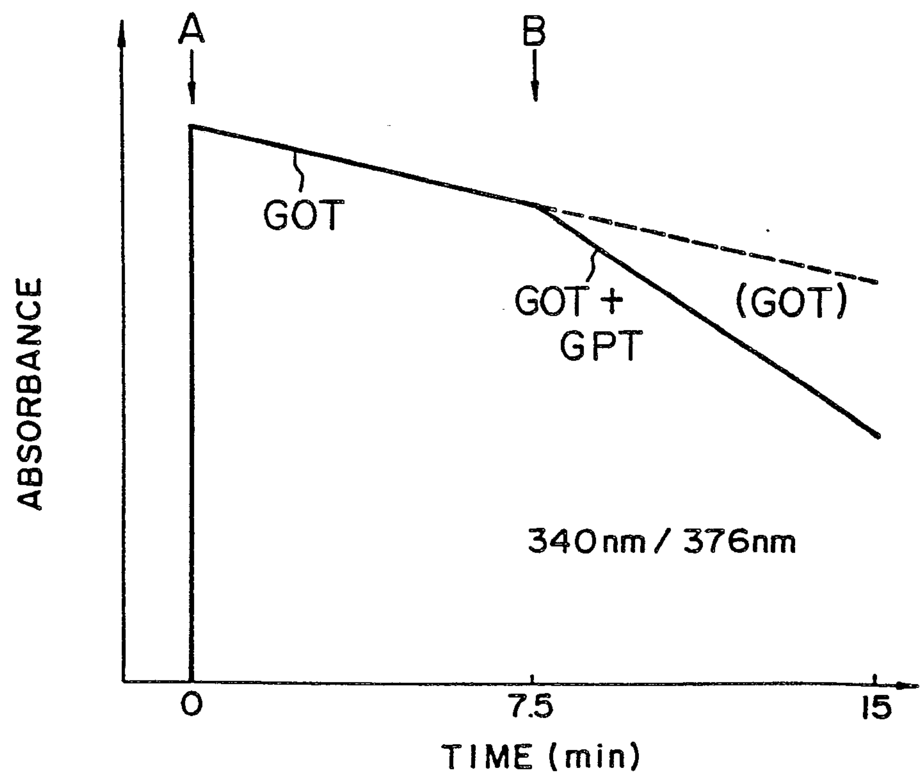
A
B
ABSORBANCE
GOT
GOT +
GPT
(GOT)
340nm / 376nm
0
7.5
15
TIME (min)